# EUROPEAN PATENT SPECIFICATION

(11) **EP 3 354 260 B1**
(45) Date of publication and mention of the grant of the patent: **09.12.2020**
(21) Application number: 17207115.1
(22) Date of filing: 04.04.2007
(51) Int. Cl.: A61K 9/127, A61K 31/7036

(54) **METHODS FOR COACERVATION INDUCED LIPOSOMAL ENCAPSULATION AND FORMULATIONS THEREOF**
VERFAHREN ZUR KOAZERVATIONSINDUZIERTE LIPOSOMALE VERKAPSELUNG UND FORMULIERUNGEN DAVON
PROCÉDÉS D'ENCAPSULATION LIPOSOMALE INDUITE PAR COACERVATION ET LEURS FORMULATIONS

(30) Priority: 06.04.2006 US 789688 P
(43) Date of publication of application: 01.08.2018
(62) Divisional of application: 07754936.8
(73) Proprietor: Insmed Incorporated, Bridgewater, NJ 08807-1704 (US)
(72) Inventor: MALININ, Vladimir, Plainsboro, NJ 08536 (US)
(74) Representative: CMS Cameron McKenna Nabarro Olswang LLP

(56) References cited:
- WO-A1-2004/002453
- WO-A2-2004/110346
- WO-A2-2007/011940
- US-A1- 2004 032 037
- FUMIYOSHI ISHII ET AL: "Procedure for Preparation of Lipid Vesicles (Liposomes) Using the Coacervation (Phase Separation) Technique", LANGMUIR, vol. 11, no. 2, 1 February 1995 (1995-02-01), pages 483-486, XP055048896, ISSN: 0743-7463, DOI: 10.1021/la00002a020

## Description

### Related Applications

This application claims the benefit of priority to U.S. Provisional Patent Application No. 60/789,688, filed April 6, 2006.

### Background of the Invention

Certain sustained release technology suitable for administration by inhalation employs lipid based formulations such as liposomes to provide prolonged therapeutic effect of an active agent and systemically by sustained release and the ability to target and enhance the uptake of the active agent into sites of disease.

WO 2004/110346 discloses lipid-based particles, e.g. liposomes, encapsulating an antiinfective for treating pulmonary infection.

For a lipid based active agent delivery system, it is often desirable to lower the lipid-to-active agent (L/A) ratio as much as possible to minimize the lipid load to avoid saturation effects in the body. For example, for lung delivery by inhalation, this may be particularly true because for chronic use, dosing of lipid could outpace clearance thus limiting the administration and thus effectiveness of the active agent product. When the active agent is a drug, a lower L/D ratio would allow more drug to be given before the dosing/clearance threshold is met.

### Summary of Invention

It is an object of the present invention to provide lipid based active agent formulations with low lipid to active agent ratios.

It is also an object of the present invention to provide a method of preparing lipid based active agent formulations with low lipid to active agent ratios.

The subject invention results from the realization that lipid based active agent formulations with low L/A ratios are achieved by preparing them using coacervation techniques.

Via methods disclosed herein, liposomes of modest size (<1 µm) comprising entrapped active agent at L/A weight ratios of typically about 0.40 - 0.49 : 1 are created. The captured volumes of liposomes have been measured, and from these numbers one is able to calculate what the theoretical entrapment should be if the active agent behaved as an ideal solute (i.e., does not interact with the liposome membrane but entraps ideally along with water). From this comparison, entrapment numbers that are 3-5X higher than expected are observed, indicating that a special interaction is occurring that allows greater than expected entrapment, and lower than expected L/A ratios. The solutions in which the liposomes form have a given active agent concentration. The concentration of active agent inside the liposomes should be about the same concentration as in the solution. However, internal active agent concentrations are calculated at least about 3X greater. It has now been discovered that this phenomenon can be explained by the formation of an active agent coacervate which initiates lipid bilayer formation around the active agent coacervate.

The present invention relates to a method of preparing a liposomal based drug formulation comprising mixing a lipid solution comprising a lipid dissolved in an organic solvent with an aqueous aminoglycoside solution to form an aminoglycoside coacervate, wherein the ratio of lipid solution flow rate to the aqueous aminoglycoside solution flow rate is 2:3, wherein the lipid is mixed with the aminoglycoside coacervate and the aminoglycoside coacervate initiates lipid bilayer formation around the aminoglycoside coacervate and wherein the lipid solution and aqueous aminoglycoside solution are mixed from two separate streams in an in-line fashion, wherein the concentration of the aminoglycoside inside the liposome is higher than the external aminoglycoside concentration. In a further embodiment the two streams enter a Y or T-connector prior to mixing in line. In a further embodiment a third stream of water or salt water is added to dilute the resulting lipid and aminoglycoside mixture. In a further embodiment the organic solvent is ethanol.

In a further embodiment, the lipid solution is added at a rate of 1-3 L/min and the aqueous aminoglycoside solution is added at a rate of 1.5-4.5 L/min. In a further embodiment, the lipid solution is added at a rate of 1 L/min and the aqueous aminoglycoside solution is added at a rate of 1.5 L/min. In a further embodiment the lipid solution is added at a rate of 1 L/min, the aqueous aminoglycoside solution is added at a rate of 1.5 L/min, and the water or salt water is added at a rate of 1 L/min.

In a further embodiment, the present invention relates to the aforementioned methods wherein the lipid is a mixture of a phospholipid and a sterol. In a further embodiment the phospholipid is dipalmitoylphosphatidylcholine (DPPC) and the sterol is cholesterol. In a further embodiment the DPPC : cholesterol ratio is 2:1 by weight. In a further embodiment, the lipid solution is at 10-30 mg/ml and the aqueous solution of the aminoglycoside is at 40-100 mg/ml. In a further embodiment the lipid solution is at 20 mg/ml and the aqueous amionoglycoside solution is at 75 mg/ml.

In a further embodiment the aminoglycoside is amikacin. In a further embodiment the aminoglycoside is tobramicin. In a further embodiment the aminoglycoside is gentamicin.

In another embodiment the lipid to aminoglycoside ratio is 0.40-0.49:1 by weight. In a further embodiment, the lipid to aminoglycoside ratio is about 0.35-0.39:1. In a further embodiment, the lipid to aminoglycoside ratio is less than 0.40:1.

In a further embodiment the lipid comprises a mixture of a phospholipid and a sterol. In a further embodiment the phospholipid is DPPC and the sterol is cholesterol. In a further embodiment the DPPC and the cholesterol is in a 2 : 1 ratio by weight.

These embodiments of the present invention, other embodiments, and their features and characteristics, will be apparent from the description, drawings and claims that follow.

### Brief Description of the Drawing

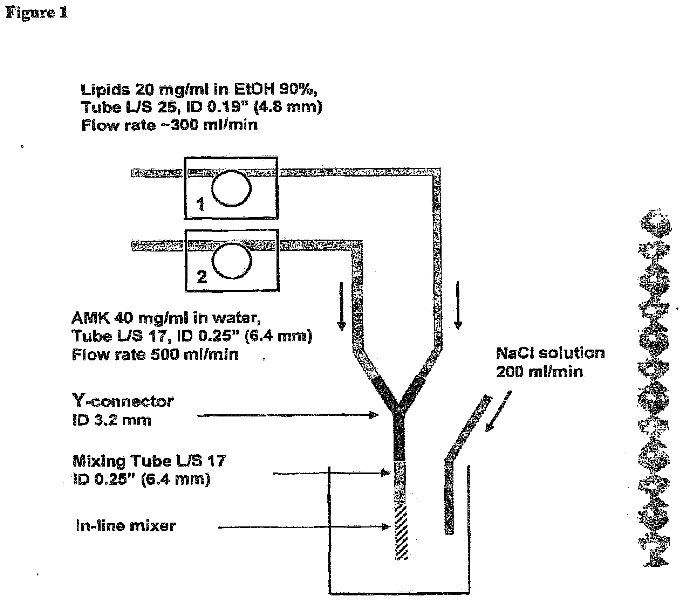

**Figure 1** depicts graphically the two-stream in-line infusion process of preparing liposomal antiinfective formulations. The flow rates depicted are non-limiting examples of flow rates subject to change as the need requires, and their ratio is not according to the invention. Also, a third NaCl solution line is depicted but this may be absent or deliver just water.
**Figure 2** depicts miscibility of amikacin sulfate with ethanol/water. Lines represent maximal amikacin concentration (base) miscible with ethanol solution at room temperature (RT) and 40°C. At higher concentrations amikacin forms a separate liquid phase (coacervates), which later precipitates as crystals. Vertical lines show ethanol concentration in the lipid/amikacin infusion mixture (300/500 parts) and after adding water 200 parts.
**Figure 3** depicts a ternary phase diagram of amikacin sulfate - water - ethanol system.
**Figure 4** depicts the effect of ionic strength and pH on ethanol-induced coacervation of BSA. A sample of BSA at 10 mg/mL in an optical cuvette was titrated with a flow of degassed ethanol under constant stirring. Light scattering signal was measured at the right angle at 600 nm wavelength using PTI fluorimeter (Photon Technology International, NJ). Temperature was fixed at 25° C.
**Figure 5** depicts the effect of MgCl₂ on ethanol induced coacervation of BSA. EtOH_{crit} is the concentration of ethanol at the onset of increase in light scattering. BSA 10 mg/mL was dissolved in NaCl 10 mM at pH 7.0.
**Figure 6** depicts the effect of low molecular weight (MW 800) polycation Polyethylenimine (PEI) on ethanol induced coacervation of BSA. BSA 10 mg/mL was dissolved in NaCl 10 mM at pH 7.0.

### Detailed Description

The present invention discloses a lipid aminoglycoside formulation prepared by forming an aminoglycoside coacervate which induces lipid bilayer formation around the aminoglycoside, as disclosed in claim 1.

The method results in low lipid to active agent ratios for the resulting lipid active agent formulation and inner active agent concentrations that are 3 to 5X higher than the external active agent concentration used. The present invention also discloses a method of preparing these lipid formulations using coacervation techniques.

### 1. Definitions

For convenience, before further description of the present invention, certain terms employed in the specification, examples and appended claims are collected here. These definitions should be read in light of the remainder of the disclosure and understood as by a person of skill in the art. Unless defined otherwise, all technical and scientific terms used herein have the same meaning as commonly understood by a person of ordinary skill in the art.

The articles "a" and "an" are used herein to refer to one or to more than one (i.e. to at least one) of the grammatical object of the article. By way of example, "an element" means one element or more than one element.

The term "bioavailable" is art-recognized and refers to a form of the subject invention that allows for it, or a portion of the amount administered, to be absorbed by, incorporated to, or otherwise physiologically available to a subject or patient to whom it is administered.

The terms "comprise" and "comprising" are used in the inclusive, open sense, meaning that additional elements may be included.

The terms "encapsulated" and "encapsulating" are refers to adsorption of active agents on the surface of lipid based formulation, association of active agents in the interstitial region of bilayers or between two monolayers, capture of active agents in the space between two bilayers, or capture of active agents in the space surrounded by the inner most bilayer or monolayer.

The term "including" is used herein to mean "including but not limited to". "Including" and "including but not limited to" are used interchangeably.

The term "lipid antiinfective formulation," or "Lip-antiinfective," or "Lip-An" discussed herein is any form of antiinfective composition where at least about 1% by weight of the antiinfective is associated with the lipid either as part of a complex with the lipid, or as a liposome where the antibiotic may be in the aqueous phase or the hydrophobic bilayer phase or at the interfacial headgroup region of the liposomal bilayer. Preferably, at least about 5%, or at least about 10%, or at least about 20%, or at least about 25%, can be so associated. Association can be measured by separation through a filter where lipid and lipid-associated antiinfective is retained and free antiinfective is in the filtrate. A "liposomal antiinfective formulation" is a lipid antiinfective formulation wherein the lipid formulation is the form of a liposome.

The term "mammal" is known in the art, and exemplary mammals include humans, primates, bovines, porcines, canines, felines, and rodents (e.g., mice and rats).

A "patient," "subject" or "host" to be treated by the subject method may mean either a human or non-human animal.

The term "pharmaceutically-acceptable salts" is art-recognized and refers to the relatively non-toxic, inorganic and organic acid addition salts of compounds, including, for example, those contained in compositions of the present invention.

The term "solvent infusion" is a process that includes dissolving one or more lipids in a small, preferably minimal, amount of a process compatible solvent to form a lipid suspension or solution (preferably a solution) and then adding the solution to an aqueous medium containing bioactive agents. Typically a process compatible solvent is one that can be washed away in a aqueous process such as dialysis. The composition that is cool/warm cycled is preferably formed by solvent infusion, with ethanol infusion being preferred. Alcohols are preferred as solvents. "Ethanol infusion," a type of solvent infusion, is a process that includes dissolving one or more lipids in a small, preferably minimal, amount of ethanol to form a lipid solution and then adding the solution to an aqueous medium containing bioactive agents. A "small" amount of solvent is an amount compatible with forming liposomes or lipid complexes in the infusion process. The term "solvent infusion" may also include an in-line infusion process where two streams of formulation components are mixed in-line.

The term "substantially free" is art recognized and refers to a trivial amount or less.

The term "surfactant" as used herein refers to a compound which lowers the surface tension of water by adsorbing at the air-water interface. Many surfactants can assemble in the bulk solution into aggregates that are known as micelles. The concentration at which surfactants begin to form micelles is known as the "critical micelle concentration" or CMC. Lipids useful for the current application may also be surfactants with extremely low CMC. Micelle-forming surfactants useful for the current application should have a CMC higher than the CMC of the lipid. At concentrations above CMC the micelle-forming surfactants can form mixed micelles composed of surfactants and lipid molecules. Upon dilution below CMC, micelle-forming surfactants will dissociate into a true solution thus leaving lipid molecules exposed to the aqueous medium. This leads to spontaneous precipitation of lipids, preferably in a form of bilayers.

The phrase "therapeutically effective amount" as used herein means that amount of a compound, material, or composition comprising a lipid drug formulation according to the present invention which is effective for producing some desired therapeutic effect by inhibiting pulmonary infections.

The term "treating" is art-recognized and refers to curing as well as ameliorating at least one symptom of any condition or disease. The term "treating" also refers to prophylactic treating which acts to defend against or prevent a condition or disease.

### 2. Coacervation

Coacervation in its simplest form can be thought of as a heaping together. In more technical terms, coacervation is the separation into two liquid phases in colloidal systems. The phase more concentrated in the colloid component (active agent) is called the coacervate, and the other phase is the equilibrium solution.

The term colloidal refers to a state of subdivision, implying that the molecules or polymolecular particles dispersed in a medium have at least in one direction a dimension roughly between 1 nm and 1 µm, or that in a system discontinuities are found at distances of that order. IUPAC Compendium of Chemical Terminology 1972, 31, 605.

A solution of macromolecules is a simple and the most common Colloid system. Small molecules also can form association colloids as reversible aggregates. An association colloid is a reversible chemical combination due to weak chemical bonding forces wherein up to hundreds of molecules or ions aggregate to form colloidal structures with sizes of from about 1 to about 2000 nanometers or larger.

Current classification of coacervation phenomenon is based on the mechanism driving the separation of two phases. Gander B, Blanco-Prieto M.J., Thomasin C, Wandrey Ch. and Hunkeler D., Coacervation/Phase Separation, In: Encyclopedia of Pharmaceutical Technology, Vol.1, Swarbrick J, Boylan J.C., Eds., Marcel Dekker, 2002, p.481-497). They include:
1. Coacervation induced by partial desolvation. This in turn can involve a binary system of a solvent and a polymer, where coacervation inducing factors are temperature or pH. Or it can be a ternary system including a solvent, a polymer, and a coacervating agent (nonsolvent for the polymer or electrolyte (salt)). This type of Coacervation is often called Simple Coacervation. Classical example of Simple Coacervation is coacervation of gelatin solution by adding alcohol (nonsolvent for gelatin). Other nonsolvents useful to induce coacervation in aqueous systems may include propanol, isopropanol, ecetone, dioxane. When electrolytes are used for polymer desolvation, the phenomenon is called salting-out. In aqueous systems the ability of ions to cause dehydration follows the Hofmeister or lyotropic series NH4⁺ < K⁺ < Na⁺ < Ca²⁺ < Mg²⁺ < Al³⁺ for cations, and Cl⁻ < SO4²⁻ < tartrate²⁻ , phosphate²⁻ < citrate³⁻, in order of increasing salting-out capacity.
2. Coacervation induced by Polymer-Polymer repulsion. In this type, the second polymer added to the solution of the first polymer induces phase separation with the 1^{st} polymer being in the coacervate phase suspended in a phase of the 2^{nd} polymer. An example of Polymer-Polymer repulsion is PLA coacervation in dichloromethane solvent induced by silicone oil.
3. Coacervation induced by non-covalent polymer cross-linking ("Complex Coacervation"). The cross-linking agent can be a polymer of opposite charge to the coacervating polymer, or di- or trivalent counter-ion to the polymer, such as Ca²⁺, Mg²⁺, Al³⁺, Zn²⁺, Tartrate²⁻ and others. Typical polymers used in complex coacervation include: polyanions Alginate, Carrageenan, Carboxymethylcellulose, Chondroitin sulfate, Cellulose sulfate, Gellan, Hyaluronic acid, Poly(acrylic acid), Xanthan; polycations Chitosan, Poly(diallyldimethylammonium chloride), Poly(L-lysine), Poly(vinylamine). In general, polyanion-polycation ineraction is controlled by a number of parameters, such as charge density, type of ionic group, chain architecture. In addition, pH, ionic strength, concentrations, temperature influence the complex formation.

Obviously, a combination of the listed above types can be used to control coacervation. Particularly, nonsolvent addition in combination with cross-linking agents, or nonsolvent and desalting agents.

In part, in the present invention, prior to coalescence, the unstable coacervate is exposed to a high concentration lipid solution. It is believed that a nucleation effect results where the coacervate seeds the precipitation of the lipids. The lipids form a bilayer encapsulating the coacervate (active agent).

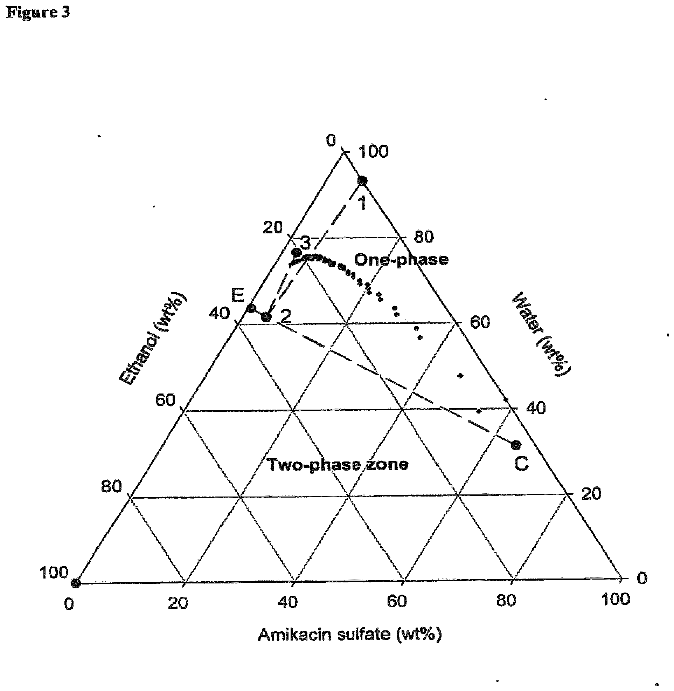

Figure 3 depicts a ternary phase diagram for an amikacin sulfate - water - ethanol system. The two-phase area under the binodial curve is a zone where the system separates into two phases, a coacervate phase and an equilibrium phase. The area above the binodial curve is a zone where single liquid phase system of amikacin sulfate dissolved in water-ethanol mixture exists. When 3 parts of amikacin sulfate solution in water at 70 mg/mL (point 1) is mixed with 2 parts of ethanol, the resulting mixture has composition (point 2), which spontaneously separates into two phases: coacervate phase rich in amikacin (point C) and equilibrium phase pour in amikacin (point E). Coacervate phase comprises only about 4.5% of total volume and originally forms as small droplets suspended in equilibrium phase. If lipids are present in surrounding solution when coacervates are just formed, they can spontaneously form bilayers around those droplets. During manufacturing it is often desired to limit exposure of the product to high ethanol concentration. For examples, another 3 parts of saline or buffer can be added consequently to the mixture, which shifts composition to the single-phase zone (point 3). Since at that point liposomes are already formed encapsulating majority of coacervate phase material, amikacin will stay encapsulated inside the liposomes.

It is key that the methods and lipid formulations of the present invention are not prepared passively, i.e encapsulation is not carried out by equilibrium alone. Coacervate formation leads to higher internal active agent concentrations relative to external active agent concentrations and lower L/A ratios.

### 3. Active Agent

The active agent coacervate can conceivably occur with any type of substance that is a biologically, physiologically, or pharmacologically active substance that acts locally or systemically in a subject. According to the present invention, the drug is an aminoglycoside (e.g., streptomycin, gentamicin, tobramycin, amikacin, netilmicin, kanamycin, and the like).

Discussion and the examples are directed primarily toward amikacin but the scope of the application is not intended to be limited to this antiinfective. Combinations of drugs can be used.

Particularly preferred aminoglycosides include amikacin, gentamicin, and tobramycin.

### 4. Lipids and Liposomes

The lipids used in the compositions prepared according to the present invention can be synthetic, semisynthetic or naturally-occurring lipids, including phospholipids, tocopherols, steroids, fatty acids, glycoproteins such as albumin, anionic lipids and cationic lipids. The lipids may be anionic, cationic, or neutral. In one embodiment, the lipid formulation is substantially free of anionic lipids. In one embodiment, the lipid formulation comprises only neutral lipids. In another embodiment, the lipid formulation is free of anionic lipids. In another embodiment, the lipid is a phospholipid. Phosholipids include egg phosphatidylcholine (EPC), egg phosphatidylglycerol (EPG), egg phosphatidylinositol (EPI), egg phosphatidylserine (EPS), phosphatidylethanolamine (EPE), and egg phosphatidic acid (EPA); the soya counterparts, soy phosphatidylcholine (SPC); SPG, SPS, SPI, SPE, and SPA; the hydrogenated egg and soya counterparts (e.g., HEPC, HSPC), other phospholipids made up of ester linkages of fatty acids in the 2 and 3 of glycerol positions containing chains of 12 to 26 carbon atoms and different head groups in the 1 position of glycerol that include choline, glycerol, inositol, serine, ethanolamine, as well as the corresponding phosphatidic acids. The chains on these fatty acids can be saturated or unsaturated, and the phospholipid can be made up of fatty acids of different chain lengths and different degrees of unsaturation. In particular, the compositions of the formulations can include dipalmitoylphosphatidylcholine (DPPC), a major constituent of naturally-occurring lung surfactant as well as dioleoylphosphatidylcholine (DOPC). Other examples include dimyristoylphosphatidylcholine (DMPC) and dimyristoylphosphatidylglycerol (DMPG) dipalmitoylphosphatidcholine (DPPC) and dipalmitoylphosphatidylglycerol (DPPG) distearoylphosphatidylcholine (DSPC) and distearoylphosphatidylglycerol (DSPG), dioleylphosphatidylethanolamine (DOPE) and mixed phospholipids like palmitoylstearoylphosphatidylcholine (PSPC) and palmitoylstearoylphosphatidylglycerol (PSPG), driacylglycerol, diacylglycerol, seranide, sphingosine, sphingomyelin and single acylated phospholipids like mono-oleoyl-phosphatidylethanol amine (MOPE).

The lipids used can include ammonium salts of fatty acids, phospholipids and glycerides, steroids, phosphatidylglycerols (PGs), phosphatidic acids (PAs), phosphotidylcholines (PCs), phosphatidylinositols (PIs) and the phosphatidylserines (PSs). The fatty acids include fatty acids of carbon chain lengths of 12 to 26 carbon atoms that are either saturated or unsaturated. Some specific examples include: myristylamine, palmitylamine, laurylamine and stearylamine, dilauroyl ethylphosphocholine (DLEP), dimyristoyl ethylphosphocholine (DMEP), dipalmitoyl ethylphosphocholine (DPEP) and distearoyl ethylphosphocholine (DSEP), N-(2, 3- di-(9 (Z)-octadecenyloxy)-prop-1-yl-N,N,N-trimethylammonium chloride (DOTMA) and 1, 2-bis(oleoyloxy)-3-(trimethylammonio)propane (DOTAP). Examples of steroids include cholesterol and ergosterol. Examples of PGs, PAs, PIs, PCs and PSs include DMPG, DPPG, DSPG, DMPA, DPPA, DSPA, DMPI, DPPI, DSPI, DMPS, DPPS and DSPS, DSPC, DPPG, DMPC, DOPC, egg PC.

Liposomal antiinfective formulations composed of phosphatidylcholines, such as DPPC, aid in the uptake by the cells in the lung such as the alveolar macrophages and helps to sustain release of the antiinfective agent in the lung (Gonzales-Rothi et al. (1991)). The negatively charged lipids such as the PGs, PAs, PSs and PIs, in addition to reducing particle aggregation, can play a role in the sustained release characteristics of the inhalation formulation as well as in the transport of the formulation across the lung (transcytosis) for systemic uptake. The sterol compounds are believed to affect the release and leakage characteristics of the formulation.

Liposomes are completely closed lipid bilayer membranes containing an entrapped aqueous volume. Liposomes can be unilamellar vesicles (possessing a single membrane bilayer) or multilamellar vesicles (onion-like structures characterized by multiple membrane bilayers, each separated from the next by an aqueous layer). The bilayer is composed of two lipid monolayers having a hydrophobic "tail" region and a hydrophilic "head" region. The structure of the membrane bilayer is such that the hydrophobic (nonpolar) "tails" of the lipid monolayers orient toward the center of the bilayer while the hydrophilic "heads" orient towards the aqueous phase. Lipid antiinfective formulations are associations lipid and the antiinfective agent. This association can be covalent, ionic, electrostatic, noncovalent, or steric. These complexes are non-liposomal and are incapable of entrapping additional water soluble solutes. Examples of such complexes include lipid complexes of amphotencin B (Janoff et al., Proc. Nat Acad. Sci., 85:6122 6126, 1988) and cardiolipin complexed with doxorubicin.

A lipid clathrate is a three-dimensional, cage-like structure employing one or more lipids wherein the structure entraps a bioactive agent.

Proliposomes are formulations that can become liposomes or lipid complexes upon corning in contact with an aqueous liquid. Agitation or other mixing can be necessary.

### 5. Methods of Preparation

The process for forming lipid active agent formulations involves a "solvent infusion" process. This is a process that includes dissolving one or more lipids in a small, preferably minimal, amount of a process compatible solvent to form a lipid solution and then infusing the solution with an aqueous medium containing the active agent. Typically a process compatible solvent is one that can be washed away in a aqueous process such as dialysis or diafiltration. "Ethanol infusion," a type of solvent infusion, is a process that includes dissolving one or more lipids in a small, preferably minimal, amount of ethanol to form a lipid solution and then infusing the solution with an aqueous medium containing the active agent. A "small" amount of solvent is an amount compatible with forming liposomes or lipid complexes in the infusion process. It is key that the conditions for the infusion process have to lead to coacervate formation. Ultimate conditions for infusing a given lipid solution with a given aqueous solution of the active agent have to be determined based on the Examples presented herein and the effect of various parameters taught below. Also useful to someone of ordinary skill in the art, are the techniques for forming coacervates as described in such references as Bunderberg de Jong, H.G., Kruyt, H.R. Koazevation (Entmischung in Kolloidalen Systemen), Koll. Zeitsch. 1930, 50(10), 39-48; Gander B, Blanco-Prieto M.J., Thomasin C, Wandrey Ch. and Hunkeler D., Coacervation/Phase Separation. In: Encyclopedia of Pharmaceutical Technology, Vol.1, Swarbrick J, Boylan J.C., Eds., Marcel Dekker, 2002, p.481-497; Newton D.W. Coacervation: Principles and Applications. In: Polymers for Controlled drug delivery. Tarcha P.J., Ed., CRC Press, Boca Raton, 1991, 67-81; Scott P.W., Williams A.C., Barry B.W., Characterization of complex coacervates of Some Tricyclic Antidepressants and evaluation of their potential for Enhancing Transdermal Flux. J. Controlled Release 1996, 41 (3), 215-227; Thomasin C., Merkle H.P., Gander B. Drug microencapsulation by PLA/PLGA Coacervation in the Light of Thermodynamics. 2. Parameters determining Microsphere Formation. J. Pharm Sci. 1998, 87 (30), 269-275; Ball V., Winterhalter M., Schwinte P., Lavalle Ph., Voegel J.-C., Schaal P. Complexation mechanism of Bovine Serum Albumin and Poly(allylamine hydrochloride). J. Phys. Chem. B. 2002, 106, 2357-2364; Mohanty B., Bohidar H.B. Systematic of Alcohol-Induced Simple Coacervation in Aqueous Gelatin Solutions. Biomacromolecules 2003,4, 1080- 1086. According to the invention, the step is performed by an in-line infusion process.

Liposome or lipid formulation sizing can be accomplished by a number of methods, such as extrusion, sonication and homogenization techniques which are well known, and readily practiced, by ordinarily skilled artisans. Extrusion involves passing liposomes, under pressure, one or more times through filters having defined pore sizes. The filters are generally made of polycarbonate, but the filters may be made of any durable material which does not interact with the liposomes and which is sufficiently strong to allow extrusion under sufficient pressure. Preferred filters include "straight through" filters because they generally can withstand the higher pressure of the preferred extrusion processes of the present invention. "Tortuous path" filters may also be used. Extrusion can also use asymmetric filters, such as Anopore™ filters, which involves extruding liposomes through a branched-pore type aluminum oxide porous filter.

Liposomes or lipid formulations can also be size reduced by sonication, which employs sonic energy to disrupt or shear liposomes, which will spontaneously reform into smaller liposomes. Sonication is conducted by immersing a glass tube containing the liposome suspension into the sonic epicenter produced in a bath-type sonicator. Alternatively, a probe type sonicator may be used in which the sonic energy is generated by vibration of a titanium probe in direct contact with the liposome suspension. Homogenization and milling apparatii, such as the Gifford Wood homogenizer, Polytron™ or Microfluidizer, can also be used to break down larger liposomes or lipid formulations into smaller liposomes or lipid formulations.

The resulting liposomal formulations can be separated into homogeneous populations using methods well known in the art; such as tangential flow filtration. In this procedure, a heterogeneously sized population of liposomes or lipid formulations is passed through tangential flow filters, thereby resulting in a liposome population with an upper and/or lower size limit. When two filters of differing sizes, that is, having different pore diameters, are employed, liposomes smaller than the first pore diameter pass through the filter. This filtrate can the be subject to tangential flow filtration through a second filter, having a smaller pore size than the first filter. The retentate of this filter is a liposomal/complexed population having upper and lower size limits defined by the pore sizes of the first and second filters, respectively.

Mayer et al. found that the problems associated with efficient entrapment of lipophilic ionizable bioactive agents such as antineoplastic agents, for example, anthracyclines or vinca alkaloids, can be alleviated by employing transmembrane ion gradients. Aside from inducing greater uptake, such transmembrane gradients can also act to increase active agent retention in the liposomal formulation.

Lipid active agent formulations have a sustained effect and lower toxicity allowing less frequent administration and an enhanced therapeutic index. In preclinical animal studies and in comparison to inhaled tobramycin (not-liposomal or lipid-based) at the equivalent dose level, liposomal amikacin was shown to have, during the time period shortly after administration to over 24 hours later, active agent levels in the lung that ranged from two to several hundred times that of tobramycin. Additionally, liposomal amikacin maintained these levels for well over 24 hours. In an animal model designed to mimic the pseudomonas infection seen in CF patients, liposomal amikacin was shown to significantly eliminate the infection in the animals' lungs when compared to free aminoglycosides.

Lung surfactant allows for the expansion and compression of the lungs during breathing. This is accomplished by coating the lung with a combination of lipid and protein. The lipid is presented as a monolayer with the hydrophobic chains directed outward. The lipid represents 80% of the lung surfactant, the majority of the lipid being phosphatidylcholine, 50% of which is dipalmitoyl phosphatidylcholine (DPPC) (Veldhuizen et al, 1998). The surfactant proteins (SP) that are present function to maintain structure and facilitate both expansion and compression of the lung surfactant as occurs during breathing. Of these, SP-B and SP-C specifically have lytic behavior and can lyse liposomes (Hagwood et al., 1998; Johansson, 1998). This lytic behavior could facilitate the gradual break-up of liposomes. Liposomes can also be directly ingested by macrophages through phagocytosis (Couveur et al., 1991; Gonzales-Roth et al., 1991; Swenson et al, 1991). Uptake of liposomes by alveolar macrophages is another means by which active agents can be delivered to the diseased site.

The lipids preferably used to form either liposomal or lipid formulations for inhalation are common to the endogenous lipids found in the lung surfactant. Liposomes are composed of bilayers that entrap the desired active agent. These can be configured as multilamellar vesicles of concentric bilayers with the active agent trapped within either the lipid of the different layers or the aqueous space between the layers. The present invention utilizes unique processes to create unique liposomal or lipid active agent formulations.

According to the present invention, the lipid aminoglycoside formulations of the present invention are prepared by an in-line infusion method where a stream of lipid solution is mixed with a stream of aminoglycoside solution in-line. For example, the two solutions may be mixed in-line inside a mixing tube preceded by a Y or T-connector. In this way, the in-line infusion method creates the best conditions for forming an active agent coacervate. This infusion method results in lower lipid to active agent ratios and higher encapsulation efficiencies.

The processes described above may be further improved by optimizing parameters such as flow rate, temperature, activation agent concentration, and salt addition after infusion step. The following experiments do not necessarily represent the methods of the present invention as indicated by the higher lipid to active agent ratios. Rather they represent a set of experiments for testing the effect of the aforementioned parameters. The multiple variables give one an idea of the novelty behind using coacervation techniques to form lipid based active agent formulations with low L/A ratios.

### 5.1 Effect of flow rates

Individual flow rates were varied while keeping the total flow rate at 800 mL/min. To do so, two separate pumps were used set at different pumping rates. The mixed solutions were infused for 10 s into a beaker containing NaCl solution such that the final NaCl concentration was 1.5% and the final ethanol concentration did not exceed 30%. After mixing, a 1 mL aliquot was run though a Sephadex G-75 gel filtration column to separate free amikacin from encapsulated. A 1 mL fraction with highest density (determined by visual turbidity) was collected for further analysis. The results are presented in Table 1. Increasing the lipid/amikacin flow rate ratio resulted in an almost constant L/D until 300/500 mL/min. With further increase of lipid rate, L/D started to increase and particle size also started getting larger. At the same time, higher lipid flow rates gave better amikacin recovery (encapsulation efficiency) as more lipid mass was added.

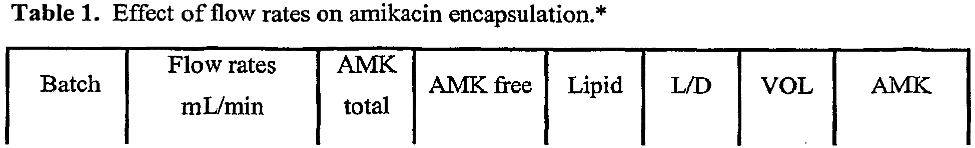

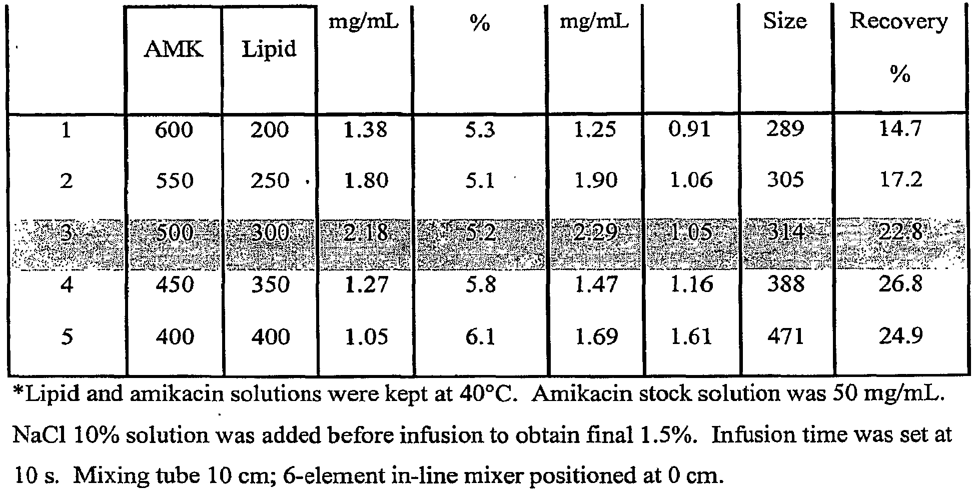

Batch 3 with the lipid/amikacin flow rates of 300/500 mL/min showed the best L/D and particle size, combined with reasonably high amikacin recovery. Thus it was decided to use these flow rates for all further experiments.

In order to reproduce the results at chosen conditions a fully washed batch (batch 6) using diafiltration was prepared as presented in Table 2. NaCl 10% solution was added into the beaker prior to infusion to make the final concentration 2% (as compared to 1.5% in the batches in Table 1). The resulting L/D (1.71) was not as good as in batch 3 in Table 1 and the particle size was higher. This may be due to an adverse effect of high NaCl concentration contacting liposomes in the early stages of liposome formation. Samples separated (washed) using gel-filtration columns tend to have better L/D than ones washed by diafiltration. This may have to do with the different degree of stress liposomes experience, or simply samples separated on the gel filtration column contained a fraction of liposomes with better L/D which does not represent the whole population.

**Table 2. Summary of the fully washed batches. Process parameters varied were: temperatures, amikacin stock concentration, and other (see Table 3 below). All batches were concentrated to nearly a maximum extent, until the inlet pressure reached 10 PSI.**

| **Batch** | **Temp, C L/AMK/W** | **AMK stock mg/mL** | **AMK total mg/mL** | **AMK free %** | **Lipid mg/mL** | **L/D** | **Size VOL nm** | **Size SD %** |
|---|---|---|---|---|---|---|---|---|
| 6 | 40/40/30 | 50 | 36.1 | 2.7 | 61.8 | 1.71 | 392 | 43.4 |
| 8 | 50/RT/30 | 50 | 48.5 | 9.6 | 49.3 | 1.02 | 332 | 32.0 |
| 9 | 50/RT/30 | 50 | 41.6 | 5.1 | 43.2 | 1.04 | 359 | 34.4 |
| 10 | 50/RT/30 | 50 | 53.1 | 10.2 | 34.4 | 0.65 | 350 | 28.6 |
| 11 | 50/RT/30 | 40 | 20.7 | 4.8 | 46.9 | 2.27 | 407 | 35.9 |
| 12 | 50/RT/30 | 40 | 81.0 | 1.9 | 49.4 | 0.61 | 341 | 33.0 |
| 13 | 50/RT/30 | 30 | 68.6 | 1.7 | 62.5 | 0.91 | 311 | 22.4 |
| 14 | 50/RT/30 | 40 | 79.6 | 1.6 | 47.8 | 0.60 | 346 | 37.2 |
| 15 | 50/RT/30 | 40 | 71.3 | 2.0 | 42.3 | 0.59 | 353 | 33.4 |
| 16 | 30/30/30 | 40 | 61.9 | 6.1 | 51.5 | 0.83 | 369 | 28.4 |
| 17 | 30/30/30 | 40 | 73.8 | 2.4 | 57.2 | 0.77 | 362 | 32.6 |
| 18 | 30/30/30 | 40 | 74.4 | 2.3 | 54.0 | 0.73 | 549 | 61.7 |

| | | | | | | | | |
|---|---|---|---|---|---|---|---|---|
| *The 3^{rd} column represents the temperatures of Lipid and Amikacin solutions just before infusion, and the temperature during washing (diafiltration). RT = room temperature. "VOL size" is the volume weighted particle size. | | | | | | | | |

**Table 3. Processing conditions for batches 1-18.***

| **Batch** | **Mixing tube cm** | **Mixer position cm** | | **NaCl added** | | **Washing conditions** | |
|---|---|---|---|---|---|---|---|
| | | | **Stock %** | **Volume parts** | **Timing to infusion** | **NaCl %** | **1st wash** |
| 1-5 | 10 | 0 | VAR | VAR | before | 1.5 | (Seph column) |
| 6 | 10 | 0 | 10 | 200 | before | 1.5 | diafiltration |
| 7 | 10 | 5 | 10 | 100 | before | 1.5 | (Seph column) |
| 8 | 10 | 5 | 10 | 150 | during | 1.5 | diafiltration |
| 9 | 10 | 5 | 10 | 150 | during | 1.5 | diafiltration |
| 10 | 10 | 5 | 10 | 100 | 5' after | 1.5 | 2x dilution |
| 11 | 10 | 5 | 10 | 150 | imm after | 1.5 | 2x dilution |
| 12 | 10 | 5 | H2O | 180 | 20" after | 1.5 | 2x dilution |
| 13 | 10 | 5 | H2O | 180 | 30" after | 1.5 | 2x dilution |
| 14 | 10 | 5 | H2O | 180 | 30" after | 1.5 | diafiltration |
| 15 | 10 | 5 | 1.5 | 180 | 30" after | 1.5 | diafiltration |
| 16 | 60 | NO | 0.9 | 180 | during | 0.9 | diafiltration |
| 17 | 60 | NO | 1.5 | 180 | during | 1.5 | diafiltration |
| 18 | 60 | 0 | 1.5 | 180 | during | 1.5 | diafiltration |

| | | | | | | | |
|---|---|---|---|---|---|---|---|
| *Lipid and amikacin solutions were infused at rates 300/500 mL/min for 30 s (examples 6 - 10) or 20 s (examples 11-18). Additional aqueous solution (NaCl or water) was added (as parts relative to 500 parts amikacin volume). | | | | | | | |

### 5.2 Effects of process temperature

The settings were kept the same as in batch 3 except that the amount of NaCl solution added was less, making the final concentration 1.0 %. Solution was added again before infusion was initiated because with the short infusion time it was difficult to make the addition during infusion. Also, during infusion the in-line mixer shifted to the end of the mixing tube under the pressure of the flow. The position of the mixer was 5 cm from the front end of the tube instead of 0 cm for batch 3. This may be important, as the L/D ratio obtained at the same temperature 40/40°C condition in batch 20 was 0.55, almost half of that in batch 3. On comparing amikacin encapsulation at different infusion temperatures, one can see that, surprisingly, lower temperatures gave better L/D. Of the temperatures tested, lipid/amikacin temperatures 30/30°C and 50/RT gave similar L/D ratios of 0.32 and 0.37. Again, as in batches 1-5, the numbers from these washed samples by gel-filtration were low, perhaps less than that if the batches had been washed by diafiltration.

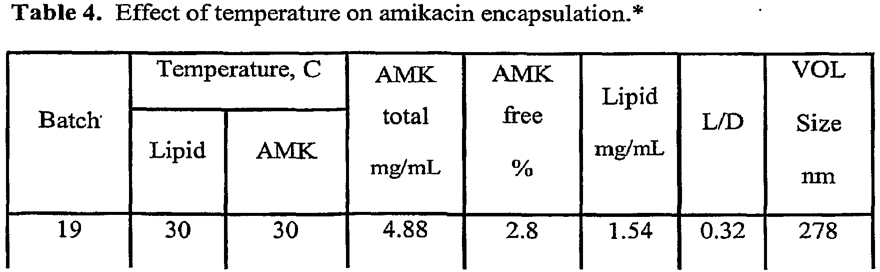

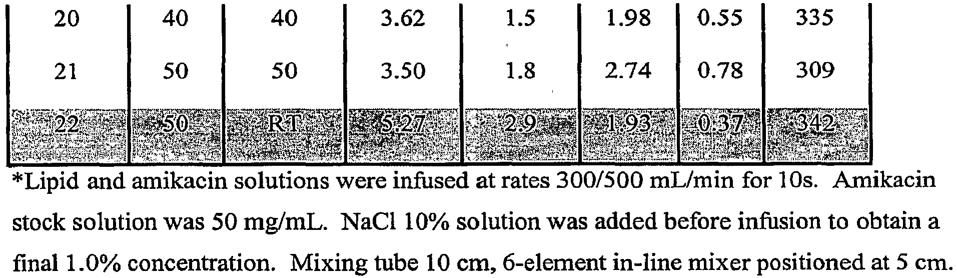

In separate experiments it was found that mixing of 90% ethanol and water at either 30°C and 30°C or 50°C and 22°C, respectively, resulted in a similar final temperature of nearly 36°C. This suggests that the temperature of the final mixture rather than that of the individual components is important for amikacin encapsulation. The temperatures 50°C/RT were used in examples 6-15. In examples 16 -18 temperatures of 30°C and 30°C for the two streams were used with comparable results, although a little less amikacin encapsulation was observed.

### 5.3 Effect of post-infusion addition of aqueous volume

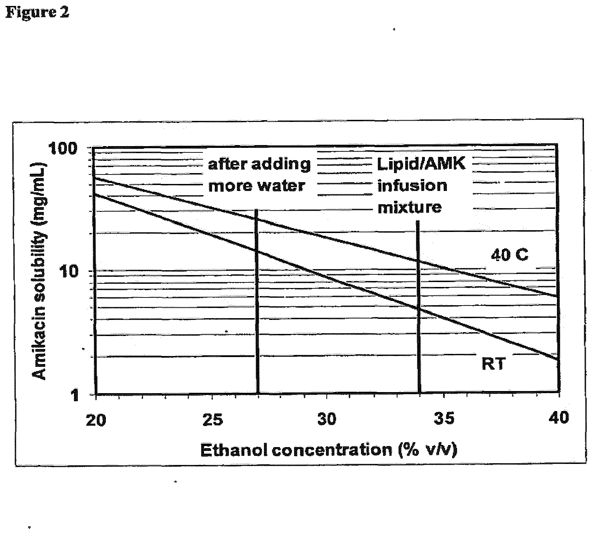

Attention was next focused on the steps of NaCl solution addition and the washing process. Process parameters were varied in various directions. Right after the infusion step at flow rates 300/500, ethanol concentration in the mixture reaches 34%. Amikacin has limited solubility at this concentration (see Figure 2).

If one starts with 50 mg/mL amikacin stock, then after mixing with the lipid solution there will be more than 30 mg/mL total amikacin where at least half (15 mg/mL) is free amikacin, assuming 50% encapsulation efficiency. This is higher than the solubility limit at 34% ethanol. One possible solution to this problem is to add more water to the vessel with the lipid/amikacin mixture, thus reducing both ethanol and amikacin concentration. For example, adding 200 parts of water (or NaCl solution) to 800 parts of lipid/amikacin would reduce ethanol to 27% (Figure 2). This makes amikacin soluble at 15 mg/mL or even higher depending on temperature.

In addition, adding NaCl may stabilize osmotic conditions. When liposomes are formed and amikacin is encapsulated at an internal concentration of 200-300 mg/mL, there is only ∼15 mg/mL or so of amikacin not encapsulated. In the absence of saline this would create an osmotic imbalance, which in turn might lead to leakage of amikacin. Adding 150 parts of 10% NaCl to 800 parts of lipid/amikacin will result in about 1.5% NaCl final concentration (outside liposomes).

A number of batches were generated where different amounts of NaCl solution (or water in some batches) were added at different times relative to the infusion event (see Table 5, compiled from Tables 2 and 3). From the table a general trend can be seen, leading to the following conclusions:
- Some time interval between infusion and addition of the aqueous volume is required to obtain lower L/D (if a short mixing tube is used). Of batches 6-15, those with an interval 20 s or longer had lower L/D. One possible explanation is that liposomes are not completely formed immediately after mixing of the streams. When a longer mixing tube is used (batches 16-18), which allows for a longer mixing time, the time interval is not required.
- Adding a high concentration NaCl solution to balance osmolality does not actually help retain amikacin. In fact, adding pure water at an appropriate time interval resulted in the lowest L/D and total amikacin concentration.
- Adding 100 parts NaCl. 10% (batch 9) 5 min after infusion gave a competitive L/D ratio but did not give as good a total amikacin concentration. It may be that NaCl, when present at early stages with relatively high ethanol concentrations, leads to increased aggregation and viscosity.

**Table 5. Role of aqueous volume and NaCl concentration added to the lipid/amikacin mixture to adjust ethanol concentration. Not all the variables shown; see Tables 2 and 3.**

| **Batch** | **AMK stock mg/mL** | **NaCl added** | | | **AMK total mg/mL** | **L/D** | **Size VOL nm** |
|---|---|---|---|---|---|---|---|
| | | **Stock %** | **Volume parts** | **Timing to infusion** | | | |
| 6 | 50 | 10 | 200 | before | 36.1 | 1.71 | 392 |
| 8 | 50 | 10 | 150 | during | 48.5 | 1.02 | 332 |
| 9 | 50 | 10 | 150 | during | 41.6 | 1.04 | 359 |
| 10 | 50 | 10 | 100 | 5' after | 53.1 | 0.65 | 350 |
| 11 | 40 | 10 | 150 | imm after | 20.7 | 2.27 | 407 |
| 12 | 40 | H₂O | 180 | 20" after | 81.0 | 0.61 | 341 |
| 13 | 30 | H₂O | 180 | 30" after | 68.6 | 0.91 | 311 |
| 14 | 40 | H₂O | 180 | 30" after | 79.6 | 0.60 | 346 |
| 15 | 40 | 1.5 | 180 | 30" after | 71.3 | 0.59 | 353 |
| 16 | 40 | 0.9 | 180 | during | 61.9 | 0.83 | 369 |
| 17 | 40 | 1.5 | 180 | during | 73.8 | 0.77 | 362 |
| 18 | 40 | 1.5 | 180 | during | 74.4 | 0.73 | 549 |

### 5.4 Effect of antiinfective stock solution

Previously it was found that using 50 mg/mL amikacin stock solution produced the best entrapment. Reducing the amikacin stock concentration to 40 mg/mL increased L/D when used in conventional processes. With the two-stream in-line infusion process, ethanol concentration reaches higher levels, so the current 50 mg/mL amikacin may not be the optimal concentration.

Table 6 summarizes the effect of using various amikacin stock concentrations. 40 mg/mL delivered comparable or better L/D values, and even improved amikacin recovery. Using less amikacin relative to a constant amount of lipid, and providing a similar L/D, resulted in a higher percent encapsulation (batch 12). Further decrease of amikacin stock concentration to 30 mg/mL resulted in a slightly increased L/D, although recovery was still impressive (batch 13).

**Table 6. Amikacin stock concentration can be reduced while improving efficiency. Amikacin recovery is calculated based on L/D obtained and assumed 100% lipid recovery.**

| **Batch** | **AMK stock mg/mL** | **AMK total mg/mL** | **AMK free %** | **Lipid mg/mL** | **L/D** | **Size VOL nm** | **AMK Recovery %** |
|---|---|---|---|---|---|---|---|
| 10 | 50 | 53.1 | 10.2 | 34.4 | 0.65 | 350 | 37.0 |
| 12 | 40 | 81.0 | 1.9 | 49.4 | 0.61 | 341 | 51.2 |
| 13 | 30 | 68.6 | 1.7 | 62.5 | 0.91 | 311 | 45.7 |
| 14 | 40 | 79.6 | 1.6 | 47.8 | 0.60 | 346 | 52.0 |

Reducing amikacin stock concentration has another implication. It reduces the concentration of free amikacin in a post-infusion lipid/amikacin mixture, allowing it to remain soluble at higher ethanol concentration. Assuming that lipid and amikacin are mixed at 300/500 ratio, amikacin stock is 50 mg/mL, and encapsulation efficiency is 37%, then initial free amikacin would be ∼20 mg/mL. Similarly, 40 mg/mL amikacin stock with 52% encapsulation would result in ∼12 mg/mL free amikacin. 30 mg/mL amikacin stock with 46% encapsulation would result in ∼10 mg/mL free amikacin.

### 6. Lipid to Active Agent Ratio

There are several ways to increase the entrapment of active agent (e.g. aminoglycosides such as amikacin, tobramycin, gentamicin) in liposomes. One way is to make very large liposomes (> 1 µm) where the entrapped volume per amount of lipid is large. This approach is not practical for inhalation (nebulization) of liposomes because 1) shear stress during nebulization tends to rupture liposomes in a size dependent manner where larger liposomes (> 0.5 µm) suffer greater release and 2) the smaller droplet sizes necessary for good lung deposition are themselves less than about ∼3 µm. So for inhalation, it is desirable to keep the liposome size as small as possible to avoid too much release. Currently, the mean diameter for the liposomes disclosed herein is less than about 0.4µm (see Table 4).

Another approach to decrease L/A is to use negatively charged lipids. The aminoglycosides listed above are highly positively charged with 4 to 5 amines per compound. Usually sulfate salts of these aminoglycosides are used in therapeutic formulations. Along with the multi-cationic character comes strong binding to negatively charged liposomes. This results in greater entrapment during liposome formation. The purpose of antiinfective formulations is to provide sustained release to the lung environment. Rapid clearance of the liposomes by macrophage uptake would run counter to this. It has been well documented that negatively charged liposomes experience a much higher degree of uptake by macrophages than neutral liposomes. Therefore, it is desirable to use neutral liposomes.

One group of technologies that allow very high active agent entrapment into small liposomes is based on gradient loading where a pH gradient, ammonium sulfate gradient, or Mg-sulfate gradient are used to load amine-drugs into liposomes: see U.S. Patent Nos.: 5,578,320 5,736,155 5,837,279 5,922,350 (pH gradient); 5,837,282 5,785,987 (Mg-sulfate gradient); and 5,316,771 (ammonium sulfate gradient). These techniques only work for membrane permeable amines (mono-amines where neutral form is permeable like doxorubicin and daunorubicin). Gradient loading will not work for the certain antiinfectives such as aminoglycosides as they are impermeable (too large and too highly charged).

All processes described herein can be easily adapted for large scale, aseptic manufacture. The final liposome size can be adjusted by modifying the lipid composition, concentration, excipients, and processing parameters.

The lipid to active agent ratio obtained by the processes of the present invention is about 0.40 to 0.49 : 1. Further, the percentage of free active agent, present after the product is dialyzed for a particular duration, is decreased.

### 7. Dosages

The dosage of any compositions of the present invention will vary depending on the symptoms, age and body weight of the patient, the nature and severity of the disorder to be treated or prevented, the route of administration, and the form of the subject composition. Any of the subject formulations may be administered in a single dose or in divided doses. Dosages for the compositions of the present invention may be readily determined by techniques known to those of skill in the art or as taught herein.

In certain embodiments, the dosage of the subject compounds will generally be in the range of about 0.01 ng to about 10 g per kg body weight, specifically in the range of about 1 ng to about 0.1 g per kg, and more specifically in the range of about 100 ng to about 50 mg per kg.

An effective dose or amount, and any possible affects on the timing of administration of the formulation, may need to be identified for any particular composition of the present invention. This may be accomplished by routine experiment as described herein, using one or more groups of animals (preferably at least 5 animals per group), or in human trials if appropriate. The effectiveness of any subject composition and method of treatment or prevention may be assessed by administering the composition and assessing the effect of the administration by measuring one or more applicable indices, and comparing the post-treatment values of these indices to the values of the same indices prior to treatment.

The precise time of administration and amount of any particular subject composition that will yield the most effective treatment in a given patient will depend upon the activity, pharmacokinetics, and bioavailability of a subject composition, physiological condition of the patient (including age, sex, disease type and stage, general physical condition, responsiveness to a given dosage and type of medication), route of administration, and the like. The guidelines presented herein may be used to optimize the treatment, e.g., determining the optimum time and/or amount of administration, which will require no more than routine experimentation consisting of monitoring the subject and adjusting the dosage and/or timing.

While the subject is being treated, the health of the patient may be monitored by measuring one or more of the relevant indices at predetermined times during the treatment period. Treatment, including composition, amounts, times of administration and formulation, may be optimized according to the results of such monitoring. The patient may be periodically reevaluated to determine the extent of improvement by measuring the same parameters. Adjustments to the amount(s) of subject composition administered and possibly to the time of administration may be made based on these reevaluations.

Treatment may be initiated with smaller dosages which are less than the optimum dose of the compound. Thereafter, the dosage may be increased by small increments until the optimum therapeutic effect is attained.

The use of the subject compositions may reduce the required dosage for any individual agent contained in the compositions (e.g., the antiinfective) because the onset and duration of effect of the different agents may be complimentary.

Toxicity and therapeutic efficacy of subject compositions may be determined by standard pharmaceutical procedures in cell cultures or experimental animals, e.g., for determining the LD₅₀ and the ED₅₀.

The data obtained from the cell culture assays and animal studies may be used in formulating a range of dosage for use in humans. The dosage of any subject composition lies preferably within a range of circulating concentrations that include the ED₅₀ with little or no toxicity. The dosage may vary within this range depending upon the dosage form employed and the route of administration utilized. For compositions of the present invention, the therapeutically effective dose may be estimated initially from cell culture assays.

### 8. Formulation

The lipid antiinfective formulations prepared according to the present invention may comprise an aqueous dispersion of liposomes. The formulation may contain lipid excipients to form the liposomes, and salts/buffers to provide the appropriate osmolarity and pH. The formulation may comprise a pharmaceutical excipient. The pharmaceutical excipient may be a liquid, diluent, solvent or encapsulating material, involved in carrying or transporting any subject composition or component thereof from one organ, or portion of the body, to another organ, or portion of the body. Each excipient must be "acceptable" in the sense of being compatible with the subject composition and its components and not injurious to the patient. Suitable excipients include trehalose, raffinose, mannitol, sucrose, leucine, trileucine, and calcium chloride. Examples of other suitable excipients include (1) sugars, such as lactose, and glucose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

### Exemplification

### Example 1

### In-Line Infusion Process

About 20 mg/ml total lipid (DPPC:cholesterol = 2:1 by wt) in ethanol and about 75 mg/ml amikacin sulfate (about 50 mg/ml amikacin) in water were mixed together into the reactor vessel by the two-stream in line infusion method. Two solutions were fed into Y-shaped connector at a rate of about 1.0 L/min and about 1.5 L/min, respectively. During the two-stream infusion, water was separately added into the reactor vessel at a similar flow rate (about 1.0 L/min) as the flow rate of lipid solution. The amikacin-lipid suspension infused into the reactor vessel is instantaneously diluted by the continuous feed of water. This additional water helps to seal the membrane by diluting ethanol and it also reduces viscosity of the suspension, consequently reducing the inlet pressure of the diafiltration cartridge. After infusion, the suspension is concentrated by reducing the volume half using diafiltration. The concentrated suspension is washed by diafiltration during a fresh supply of 3.0% NaCl solution. The washed suspension is further concentrated by diafiltration until the desired total amikacin concentration is achieved. The results are given in Table 7.

**Table 7.**

| Lot | Washing Temp (°C) | Total [amikacin] mg/ml | Total [lipid] mg/ml | Lipid/Drug |
|---|---|---|---|---|
| I | RT* | 130.3 | 54.2 | 0.42 |
| II | RT* | 126.0 | 57.0 | 0.45 |
| III | 35 | 130.0 | 60.9 | 0.47 |

| | | | | |
|---|---|---|---|---|
| *Room temperature (19∼23 °C). | | | | |

### Example 2

### Encapsulation of Bovine Serum Albumin (BSA) by coacervation technique (not according to the invention)

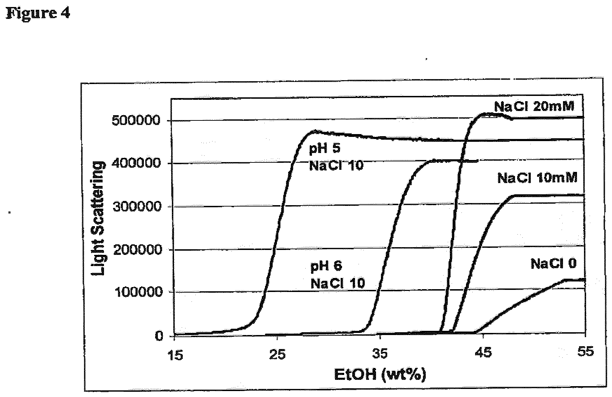

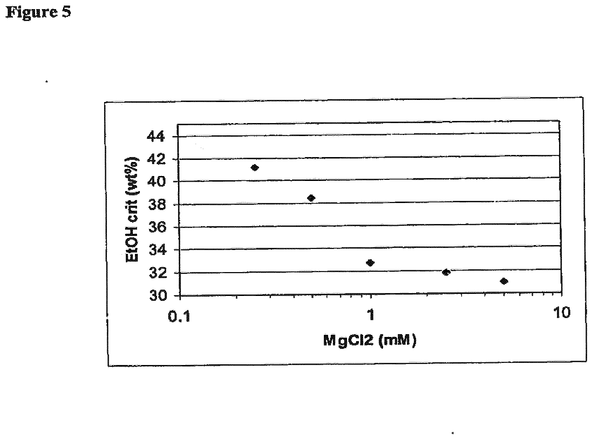

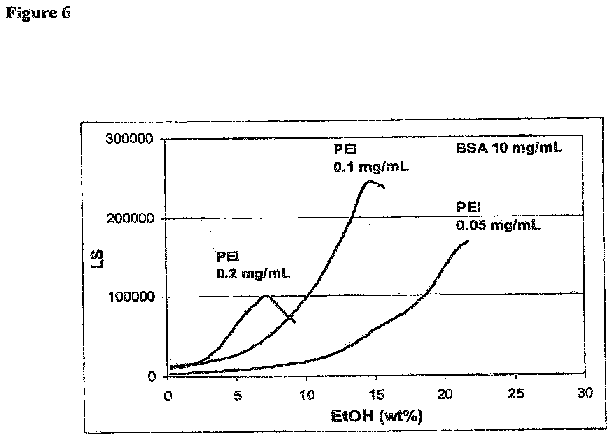

BSA is a protein having isoelectric point pI = 4.9. At pH above that point, it can be considered as a colloid with a net negative charge. It has been shown to form complex coacervates with various polyelectrolytes, such as Poly(allylamine hydrochloride), which in turn is affected by the medium ionic strength, pH and temperature. It was found that addition of nonsolvent to albumin (ethanol) can also induce coacervation. When BSA is dissolved in water at pH 7.0, and ethanol concentration added exceeds ∼45 wt%, BSA molecules aggregate to form droplets of coacervate phase thus leading to strong increase in light scattering. Adding NaCl (increasing ionic strength) results in less ethanol needed to induce coacervation. Lowering the pH has a similar effect (Figure 4). Di-valent ions (e.g. Mg²⁺) have an even stronger effect on lowering the critical ethanol concentration required to induce BSA coacervation (Figure 5). The most drastic effect was found when the low molecular weight polycation PEI was added to the BSA solution (Figure 6). Thus, 0.05 mg/mL of PEI in molar terms is ∼60 µM concentration, which represents only about 1 molecule PEI per 3 molecules of BSA.

To encapsulate BSA into liposomes, a BSA aqueous solution at 10 mg/ml in 20 mM NaCl, pH 5.5 was used. A lipid solution was prepared separately at a concentration of 10 mg/mL and a molar ratio DPPC/DPPG/Cholesterol of 60:5:40 in 95 % ethanol. All solutions were preheated to 30° C. The lipid solution (0.4 mL) was added by pipette into a 1 mL BSA solution in a test tube and immediately vortexed to ensure complete mixing. 20 seconds later 0.6 mL of 5% sucrose solution was added and vortexing repeated. To determine BSA encapsulation, 0.8 mL of the resulting liposome suspension was placed on 5-20% sucrose gradient and centrifuged 30 min at 30,000 RPM. The loaded liposomes formed a pellet heavier than 20% sucrose. The pellet was collected and quantitated for lipids and BSA. Lipids were measured by reverse-phase HPLC and BSA was measured by fluorescence (excitation 280 nm, emission 320 nm). It was found that the pellet contained 1.6 mg lipid and 1.3 mg BSA thus giving L/D ratio of 1.2 which is lower than what is normally seen for proteins (for example, see U.S. Patent No. 6,843,942, where encapsulation of recombinant human superoxide dismutase (rh-SOD) in a DPPC-cholesterol-stearylamine formulation was prepared with a L/D ratio of ∼5).

### References

1. Veldhuizen, R., Nag, K., Orgeig, S. and Possmayer, F., The Role of Lipids in Pulmonary Surfactant, Biochim. Biophys. Acta 1408:90-108 (1998).
2. Hagwood, S., Derrick, M. and Poulain, F., Structure and Properties of Surfactant Protein B, Biochim. Biophys. Acta 1408:150-160 (1998).
3. Johansson, J., Structure and Properties of Surfactant ProteinC, Biochim. Biophys. Acta 1408:161-172 (1998).
4. Ikegami, M. and Jobe, A.H., Surfactant Protein Metabolism in vivo, Biochim. Biophys. Acta 1408:218-225 (1998).
5. Couveur, P., Fattel, E. and Andremont,A., Liposomes and Nanoparticles in the Treatment of Intracellular Bacterial Infections, Pharm. Res. 8:1079-1085 (1991).
6. Gonzales-Rothi, R.J., Casace, J., Straub, L., and Schreier, H., Liposomes and Pulmonary Alveolar Macrophages: Functional and Morphologic Interactions, Exp. Lung Res. 17:685-705 (1991).
7. Swenson, C.E., Pilkiewicz, F.G., and Cynamon, M.H., Liposomal Aminoglycosides and TLC-65 Aids Patient Care 290-296 (Dec., 1991).
8. Costerton, J.W., Stewart, P.S., and Greenberg, E.P., Bacterial Biofilms: A Common Cause of Persistent Infections, Science 284:1318-1322 (1999).
9. Cash, H.A., Woods, D.E., McCullough, W.G., Johanson, J.R., and Bass, J.A., A Rat Model of Chronic Respiratory Infection with Pseudomonas aeruginosa, American Review of Respiratory Disease 119:453-459 (1979).
10. Cantin, A.M. and Woods, D.E. Aerosolized Prolastin Suppresses Bacterial Proliferation in a Model of Chronic Pseudomonas aeruginosa Lung Infection, Am. J. Respir. Crit. Care Med. 160:1130-1135 (1999).
11. Price, K.E., DeFuria, M.D., Pursiano, T.A. Amikacin, an aminoglycoside with marked activity against antibiotic-resistant clinical isolates. J Infect Dis 134:S249261(1976).
12. Damaso, D., Moreno-Lopez, M., Martinez-Beltran, J., Garcia-Iglesias, M.C. Susceptibility of current clinical isolates of Pseudomonas aeruginosa and enteric gram-negative bacilli to Amikacin and other aminoglycoside antibiotics. J Infect Dis 134:S394-90 (1976).
13. Pile, J.C., Malone, J.D., Eitzen, E.M., Friedlander, A.M., Anthrax as a potential biological warfare agent. Arch. Intern. Med. 158:429-434 (1998).
14. Gleiser, C.A., Berdjis, C.C., Hartman, H.A., & Glouchenour, W.S., Pathology of experimental respiratory anthrax in Macaca mulatta. Brit. J. Exp. Path., 44:416-426 (1968).

## Claims

1. A method of preparing a liposomal based drug formulation comprising mixing a lipid solution comprising a lipid dissolved in an organic solvent with an aqueous aminoglycoside solution to form an aminoglycoside coacervate,
wherein the ratio of lipid solution flow rate to the aqueous aminoglycoside solution flow rate is 2:3,
wherein the lipid is mixed with the aminoglycoside coacervate and the aminoglycoside coacervate initiates lipid bilayer formation around the aminoglycoside coacervate and
wherein the lipid solution and aqueous aminoglycoside solution are mixed from two separate streams in an in-line fashion, wherein the concentration of the aminoglycoside inside the liposome is higher than the external aminoglycoside concentration.

2. The method of claim 1, wherein the lipid solution is provided at a flow rate of 1 L/min to 3 L/min and the aqueous aminoglycoside solution is provided at a flow rate of 1.5 L/min to 4.5 L/min.

3. The method of claim 1, wherein the two streams enter a Y or T-connector prior to mixing inline.

4. The method of claim 1 or 3, wherein a third stream of water or salt water is added to dilute the resulting lipid and aminoglycoside mixture.

5. The method of claim 2, wherein the lipid solution has a flow rate of 1 L/min and the aqueous aminoglycoside solution has a flow rate of 1.5 L/min.

6. The method of claim 1, 3 or 4 wherein the organic solvent is ethanol.

7. The method of claim 1, 3, 4 or 6, wherein the lipid solution comprises a mixture of a phospholipid and a sterol.

8. The method of claim 7, wherein the phospholipid is dipalmitoylphosphatidylcholine (DPPC) and the sterol is cholesterol.

9. The method of claim 8, wherein the DPPC: cholesterol ratio is 2:1 by weight.

10. The method of claim 1, 3, 4 or 6-9, wherein the concentration of the lipid in the lipid solution is 10-30 mg/ml and the concentration of the aminoglycoside in the aqueous aminoglycoside solution is 40-100 mg/ml.

11. The method of claim 1, 3, 4 or 6-10 wherein the aminoglycoside is amikacin.

12. The method of claim 1, wherein the aminoglycoside is amikacin, tobramicin, or gentamicin.

13. The method of claim 11, wherein the amikacin is amikacin sulfate.

## Patentansprüche

1. Verfahren zum Herstellen einer Arzneimittelformulierung auf Liposomenbasis, die ein Mischen einer Lipidlösung umfasst, die ein in einem organischen Lösungsmittel mit einer wässrigen Aminoglykosidlösung gelöstes Lipid umfasst, um ein Aminoglykosid-Koazervat auszubilden, wobei das Verhältnis der Flussrate der Lipidlösung zu der Flussrate der wässrigen Aminoglykosidlösung 2:3 beträgt, wobei das Lipid mit dem Aminoglykosid-Koazervat gemischt wird und das Aminoglykosid-Koazervat eine Lipiddoppelschichtbildung um das Aminoglykosid-Koazervat herum einleitet, und wobei die Lipidlösung und die wässrige Aminoglykosidlösung aus zwei getrennten Strömen in einer Inline-Art gemischt werden, wobei die Konzentration des Aminoglykosids innerhalb des Liposoms höher als die externe Aminoglykosidkonzentration ist.

2. Verfahren nach Anspruch 1, wobei die Lipidlösung mit einer Flussrate von 1 l/min bis 3 l/min bereitgestellt wird und die wässrige Aminoglykosidlösung mit einer Flussrate von 1,5 l/min bis 4,5 l/min bereitgestellt wird.

3. Verfahren nach Anspruch 1, wobei die beiden Ströme in einen Y- oder T-Verbinder vor dem Inline-Mischen eindringen.

4. Verfahren nach Anspruch 1 oder 3, wobei ein dritter Strom aus Wasser oder Salzwasser zugesetzt wird, um die resultierende Lipid- und Aminoglykosid-Mischung zu verdünnen.

5. Verfahren nach Anspruch 2, wobei die Lipidlösung eine Flussrate von 1 l/min aufweist und die wässrige Aminoglykosidlösung eine Flussrate von 1,5 l/min aufweist.

6. Verfahren nach Anspruch 1, 3 oder 4, wobei das organische Lösungsmittel Ethanol ist.

7. Verfahren nach Anspruch 1, 3, 4 oder 6, wobei die Lipidlösung eine Mischung aus einem Phospholipid und einem Sterol umfasst.

8. Verfahren nach Anspruch 7, wobei das Phospholipid Dipalmitoylphosphatidylcholin (DPPC) ist und das Sterol Cholesterol ist.

9. Verfahren nach Anspruch 8, wobei das DPPC:Cholesterol-Verhältnis 2:1 nach Gewicht beträgt.

10. Verfahren nach Anspruch 1, 3, 4 oder 6-9, wobei die Konzentration des Lipids in der Lipidlösung 10-30 mg/ml beträgt und die Konzentration des Aminoglykosids in der wässrigen Aminoglykosidlösung 40-100 mg/ml beträgt.

11. Verfahren nach Anspruch 1, 3, 4 oder 6-10, wobei das Aminoglykosid Amikacin ist.

12. Verfahren nach Anspruch 1, wobei das Aminoglykosid Amikacin, Tobramycin oder Gentamicin ist.

13. Verfahren nach Anspruch 11, wobei das Amikacin Amikacinsulfat ist.

## Revendications

1. Procédé de préparation d'une formulation de médicament à base de liposomes comprenant le mélange d'une solution lipidique comprenant un lipide dissous dans un solvant organique avec une solution d'aminoglycoside aqueuse pour former un coacervat d'aminoglycoside,
dans lequel le rapport entre le débit de solution lipidique et le débit de solution d'aminoglycoside aqueuse est de 2:3,
dans lequel le lipide est mélangé avec le coacervat d'aminoglycoside et le coacervat d'aminoglycoside lance la formation d'une bicouche lipidique autour du coacervat d'aminoglycoside, et
dans lequel la solution lipidique et la solution d'aminoglycoside aqueuse sont mélangées à partir de deux flux séparés en ligne, dans lequel la concentration d'aminoglycoside à l'intérieur du liposome est supérieure à la concentration d'aminoglycoside externe.

2. Procédé selon la revendication 1, dans lequel la solution lipidique est fournie à un débit de 1 à 3 L/min et la solution d'aminoglycoside aqueuse est fournie à un débit de 1,5 à 4,5 L/min.

3. Procédé de la revendication 1, dans lequel les deux flux entrent dans un raccord Y ou T avant de se mélanger en ligne.

4. Procédé selon la revendication 1 ou 3, dans lequel un troisième flux d'eau ou d'eau salée est ajouté pour diluer le mélange de lipide et d'aminoglycoside résultant.

5. Procédé selon la revendication 2, dans lequel la solution lipidique a un débit de 1 L/min et la solution d'aminoglycoside aqueuse a un débit de 1,5 L/min.

6. Procédé selon la revendication 1, 3 ou 4, dans lequel le solvant organique est de l'éthanol.

7. Procédé selon la revendication 1, 3, 4 ou 6, dans lequel la solution lipidique comprend un mélange d'un phospholipide et d'un stérol.

8. Procédé de la revendication 7, dans lequel le phospholipide est la dipalmitoylphosphatidylcholine (DPPC) et le stérol est le cholestérol.

9. Procédé de la revendication 8, dans lequel, dans la DPPC : le taux de cholestérol est de 2:1 en poids.

10. Procédé selon la revendication 1, 3, 4 ou 6 à 9, dans lequel la concentration du lipide dans la solution lipidique est de 10 à 30 mg/ml et la concentration de l'aminoglycoside dans la solution d'aminoglycoside aqueuse est de 40 à 100 mg/ml.

11. Procédé selon la revendication 1, 3, 4 ou 6 à 10, dans lequel l'aminoglycoside est l'amikacine.

12. Procédé selon la revendication 1, dans lequel l'aminoglycoside est l'amikacine, la tobramicine ou la gentamicine.

13. Procédé selon la revendication 11, dans lequel l'amikacine est le sulfate d'amikacine.
